Europäisches Patentamt

European Patent Office

Office européen des brevets

(11) Veröffentlichungsnummer : **0 060 986**
**B1**

(12)                          EUROPÄISCHE PATENTSCHRIFT

(45) Veröffentlichungstag der Patentschrift :
26.09.84

(21) Anmeldenummer : 82100919.8

(22) Anmeldetag : 09.02.82

(51) Int. Cl.³ : **A 61 B   3/08**

(54) **Sehprobe zur Prüfung der binokularen Lesefähigkeit.**

(30) Priorität : 20.02.81 DE 8104761 U

(43) Veröffentlichungstag der Anmeldung :
29.09.82 Patentblatt 82/39

(45) Bekanntmachung des Hinweises auf die Patenterteilung : 26.09.84 Patentblatt 84/39

(84) Benannte Vertragsstaaten :
FR GB IT NL

(56) Entgegenhaltungen :
GB-A-   264 615
GB-A-   682 350
US-A- 2 280 297

(73) Patentinhaber : **Firma Carl Zeiss**
**D-7920 Heidenheim (Brenz) (DE)**

(72) Erfinder : **Gottlob, Heinz**
**Finkenweg 35**
**D-7923 Königsbronn (DE)**
Erfinder : **Falk, Horst, Dipl.-Phys.**
**Schleiermacherstrasse 3**
**D-7080 Aalen (DE)**

## Beschreibung

Die vorliegende Erfindung betrifft eine zur Prüfung der binokularen Lesefähigkeit dienende Sehprobe, zu deren Betrachtung vor den Augen des Prüflings Polarisationsfilter mit gegeneinander gekreuzten Schwingungsrichtungen anzuordnen sind, gemäß dem Oberbegriff des Anspruchs 1. Eine derartige Sehprobe ist aus der US-A 682 350 bekannt.

Es sind derartige Sehproben bekannt, die aus zwei Teilen zusammengesetzte Figuren enthalten, wobei die Figurenteile polarisierend mit gekreuzt zueinander verlaufenden Polarisationsrichtungen ausgebildet sind. Der Prüfling sieht deshalb mit jedem Auge nur einen Teil der Figur. Bei normaler binokularer Lesefähigkeit erscheinen diese beiden optisch getrennten Seheindrücke dem Prüfling als Gesamtfigur.

Bekannt sind auch Sehproben, die in zwei horizontalen Zeilen je zwei oder mehr Buchstaben enthalten. Die Buchstaben jeder Zeile sind so polarisierend mit gekreuzt zueinander verlaufenden Polarisationsrichtungen ausgebildet, daß übereinanderstehende Buchstaben ebenfalls gekreuzt verlaufende Polarisationsrichtungen aufweisen. Der Prüfling sieht bei normaler Sehfähigkeit vier oder mehr Buchstaben.

Alle diese Sehproben erfordern eine gewisse Konzentration des Prüflings, da er das von ihm wahrgenommene Bild beschreiben muß. Der Prüfer kann nur aus dieser Beschreibung seine Schlüsse ziehen.

Der vorliegenden Erfindung liegt nun die Aufgabe zugrunde, eine Sehprobe zur Prüfung der binokularen Lesefähigkeit zu schaffen, welche es dem Prüfenden ermöglicht sofort zu erkennen, ob beim Prüfling die binokulare Lesefähigkeit gegeben ist und die zudem eine Prüfung der Sehschärfe ermöglicht.

Diese Aufgabe wird bei einer Sehprobe nach dem Oberbegriff des Anspruchs 1 dadurch gelöst, daß auf einem Träger mehrere Folien mit jeweils verschieden großen Buchstabenzeilen austauschbar angeordnet sind, daß auf jeder eine einheitliche Buchstabengröße aufweisenden Folie ober- und unterhalb einer ersten, aus Licht nicht-polarisierend reflektierenden Buchstaben gebildeten Zeile jeweils mindestens eine, aus Licht in einer einheitlichen Polarisationsrichtung reflektierenden Buchstaben gebildete Zeile angeordnet ist, und daß die Polarisationsrichtungen aller ein einheitliches Testsymbol bildender Buchstaben in den ober- und unterhalb der ersten Zeile liegenden Zeilen gekreuzt zueinander verlaufen.

Besonders vorteilhaft ist es den Buchstaben einer Zeile eine einheitliche Polarisationsrichtung zu geben. Es ist jedoch auch möglich die Zeilen so auszubilden, daß jeweils aufeinanderfolgende Buchstaben gekreuzte Polarisationsrichtungen aufweisen.

Bei dieser Sehprobe wird die mittlere Zeile von beiden Augen des Prüflings wahrgenommen. Sie dient deshalb als ein Verriegelungsmittel, das die Augen des Prüflings auf den Text fixiert. Werden vom Prüfling alle Zeilen mit demselben Kontrast gesehen, so liegt einwandfreies binokulares Lesevermögen vor. Dieses ist gestört, wenn die Textzeilen mit verschiedenem Kontrast wahrgenommen werden; es ist nicht vorhanden, wenn der Prüfling zusätzlich zur mittleren Zeile nur die Hälfte des Textes wahrnimmt.

Zweckmäßig stellen die Buchstabenzeilen einen fortlaufenden Text dar, so daß der Prüfende aus dem vom Prüfling vorgelesenen Text sofort entnehmen kann, ob binokulares Seh- und Lesevermögen vorliegt.

Die Buchstaben mehrerer Folien können aus unterschiedlichen Drucktypen gebildet sein und der zu lesende Text kann bezüglich Inhalt und Sprache gestaltet werden.

Die neue Sehprobe eignet sich insbesondere für die Nahprüfung, wobei in einfacher Weise durch Wechsel der Folien dem Prüfling Buchstaben verschiedener Größe dargeboten und dabei festgestellt werden kann, ab welcher Buchstabengröße der Text gelesen werden kann. Zugleich läßt sich überprüfen, ob und in welcher Weise sich beide Augen beim Lesen beteiligen.

Zur Nahprüfung ist die Sehprobe zweckmäßig so ausgestaltet, daß die Folien, welche die Buchstaben- bzw. Textreihen tragen umschlagbar auf einem, als Pult ausgebildeten Träger angeordnet sind, der mindestens eine Fläche mit einer zum Nah-Sehen geeigneter Schräglage aufweist.

Die neue Sehprobe kann besonders vorteilhaft dazu verwendet werden festzustellen wie weit man bei Sehbinderten mit vergrößernden Sehhilfen beliebiger Art, beispielsweise Lupenbrillen binokulares Sehen erreichen kann.

Die Erfindung wird im folgenden anhand der Figuren 1-4 der beigefügten Zeichnungen näher erläutert. Im einzelnen zeigen:

Figur 1 eine Prinzipdarstellung eines Ausführungsbeispiels;

Figur 2 den monokularen Seheindruck für das linke Auge;

Figur 3 den monokularen Seheindruck für das rechte Auge;

Figur 4 den Seheindruck beim Binokularsehen.

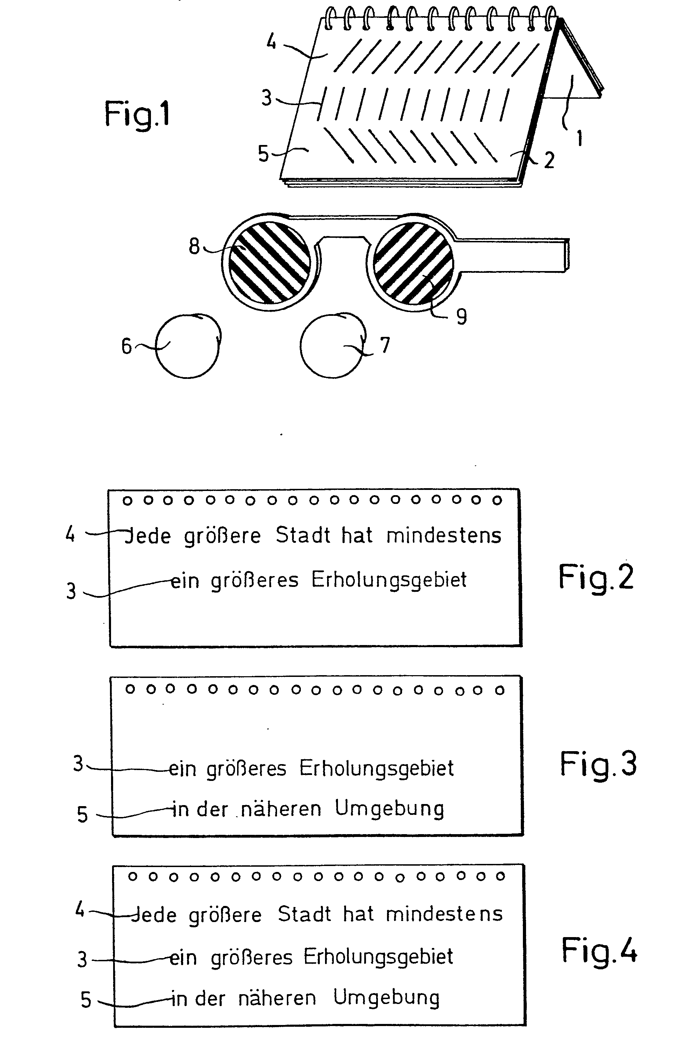

In Fig. 1 ist mit 1 ein pultförmig ausgebildeter Träger bezeichnet, der mehrere umschlagbar angeordnete Folien 2 trägt. Die sichtbare oberste Folie 2 enthält auf weißem Untergrund drei Buchstabenzeilen 3, 4, 5. Die mittlere Zeile 3 besteht aus nicht-polarisierenden Buchstaben, während die Zeilen 4 und 5 aus polarisierenden Buchstaben bestehen, die beispielsweise in einem Druckverfahren oder mit Hilfe von Fototechnik hergestellt sind. Die schematisch angedeuteten Polarisationsrichtungen sind innerhalb der Zeilen 4 und 5 einheitlich, jedoch verlaufen die Polarisationsrichtungen der Zeilen 4 und 5 gekreuzt zueinander.

Es ist auch möglich die Zeilen 4 und 5 so auszubilden, daß aufeinander folgende Buchstaben gekreuzte Polarisationsrichtungen aufweisen.

Vor den Augen 6, 7 des Prüflings sind Polarisationsfilter 8, 9 angeordnet, die gegeneinander gekreuzte Schwingungsrichtungen aufweisen. Damit sind im gezeigten Beispiel für das linke Auge 6 nur die Zeilen 3, 4 wahrnehmbar, während dies für das rechte Auge 7 die Zeilen 3, 5 sind. Ist das binokulare Lesevermögen des Prüflings voll ausgebildet, so sieht er beidäugig die Buchstabenzeilen 3, 4, 5 mit gleichem Kontrast. Diese Buchstabenzeilen stellen einen zusammenhängenden Text dar, sodaß der Prüfende schnell und einfach erkennen kann, ob der den wahrgenommenen Text ablesende Prüfling binokular sieht.

Im dargestellten Ausführungsbeispiel ist der pultförmige Träger 1 so ausgebildet, daß seine, die sichtbaren Folien 2 tragende Fläche eine zum Nah-Sehen geeignete Schräglage aufweist.

Die Folien 2 sind umschlagbar auf dem Träger 1 befestigt und enthalten verschieden große Buchstabenreihen. Damit läßt sich einfach feststellen, ab welcher Größe die Texte vom Prüfling gelesen werden können.

Fig. 2 zeigt den monokularen Seheindruck für das linke Prüflingsauge 6, das den zusammenhängenden Text in den Zeilen 3, 4 wahrnimmt. Den monokularen Seheindruck des rechten Auges zeigt Fig. 3; hier werden die Zeilen 3, 5 wahrgenommen. Beim binokularen Sehen wird das in Fig. 4 gezeigte Bild wahrgenommen, das aus den Zeilen 3, 4, 5 besteht und den gesamten Text wiedergibt.

## Ansprüche

1. Sehprobe zur Prüfung der binokularen Lesefähigkeit mit auf einer Fläche in mehreren Zeilen angeordneten, Licht nicht-polarisierenden oder polarisierenden Buchstaben, zu deren Betrachtung vor den Augen des Prüflings Polarisationsfilter mit gegeneinander gekreuzten Schwingungsrichtungen anzuordnen sind, dadurch gekennzeichnet, daß auf einem Träger (1) mehrere Folien (2) mit jeweils verschieden großen Buchstabenzeilen austauschbar angeordnet sind, daß auf jeder eine einheitliche Buchstabengröße aufweisenden Folie (2) ober- und unterhalb einer ersten, aus Licht nicht-polarisierend reflektierenden Buchstaben gebildeten Zeile (3) jeweils mindestens eine, aus Licht in einer einheitlichen Polarisationsrichtung reflektierenden Buchstaben gebildete Zeile (4, 5) angeordnet ist, und daß die Polarisationsrichtungen aller ein einheitliches Testsymbol bildender Buchstaben in den ober- und unterhalb der ersten Zeile (3) liegenden Zeilen (4, 5) gekreuzt zueinander verlaufen.

2. Sehprobe nach Anspruch 1 dadurch gekennzeichnet, daß die Buchstaben einer Zeile (4 bzw. 5) eine einheitliche Polarisationsrichtung haben.

3. Sehprobe nach Anspruch 1, dadurch gekennzeichnet, daß in jeder der aus polarisierenden Buchstaben gebildeten Zeile (4, 5) aufeinanderfolgende Buchstaben gekreuzte Polarisationsrichtungen haben.

4. Sehprobe nach Anspruch 1 und 2 oder 3, dadurch gekennzeichnet, daß die Folien (2) umschlagbar auf einem Träger (1) angeordnet sind.

5. Sehprobe nach Anspruch 4, dadurch gekennzeichnet, daß der Träger (1) als Pult ausgebildet ist, der mindestens eine Fläche mit einer zum Nah-Sehen geeigneten Schräglage aufweist.

## Claims

1. Optical test chart for testing binocular reading ability, consisting of letters which do not polarize or which polarize light and which are arranged on one surface in several lines, for the viewing of said letters polarizing filters with oppositely crossed directions of vibration have to be arranged in front of the eyes of the test subject, characterized in that a plurality of foils (2) bearing lines of letters of different size respectively are arranged interchangeably on a support (1), that each foil (2) contains letters of the same size arranged in a first line (3) consisting of reflecting letters which do not polarize light and in at least one line (4) arranged above and on line (5) arranged below said first line (3), each of said lines consisting of letters reflection light in the same polarizing direction, and that the polarizing directions of all letters forming a uniform test symbol in the lines (4, 5) above and below said first line (3) are crossed with respect to each other.

2. Optical test chart according to claim 1, characterized in that the letters of one line (4 or 5) have the same direction of polarization.

3. Optical test chart according to claim 1, characterized in that in each of the lines (4, 5) consisting of polarizing letters successive letters have crossed directions of polarization.

4. Optical test chart according to claim 1 and 2, or 3, characterized in that the foils (2) are arranged in turnable manner on a support (1).

5. Optical test chart according to claim 4, characterized in that the support (1) is developed as a lectern which has at least one surface with an oblique position which is suitable for near-vision.

## Revendications

1. Optotype pour l'examen de la capacité de lecture binoculaire, possédant, sur une surface, des lettres disposées sur plusieurs lignes, polarisant ou ne polarisant pas la lumière, pour l'observation desquelles sont disposés devant les yeux du sujet des filtres polarisants ayant des directions de vibration croisées l'une par rapport à l'autre, caractérisé en ce que plusieurs feuilles (2), ayant chacune des lignes de lettres de différentes tailles, sont disposées sur un support (1) d'une manière interchangeable ; que, sur chaque feuille (2), au-dessus et au-dessous d'une première ligne (3) formée de lettres réfléchissant la lumière sans polarisation, est placée au moins une ligne (4, 5) formée de lettres réfléchissant la lumière selon une direction de polarisation uniforme ; et que les directions de polarisation de toutes les lettres formant un symbole d'essai uniforme sont croisées les unes par rapport aux autres dans les lignes (4, 5) se trouvant au-dessus et au-dessous de la première ligne (3).

2. Optotype selon la revendication 1, caractérisé en ce que les lettres d'une ligne (4 ou 5) ont une direction de polarisation uniforme.

3. Optotype selon la revendication 1, caractérisé en ce que, sur chacune des lignes (4, 5) formées de lettres polarisantes, des lettres successives présentent des directions de polarisation croisées.

4. Optotype selon la revendication 1 et 2 ou 3, caractérisé en ce que les feuilles (2) sont disposées sur un support (1) d'une manière rabattable.

5. Optotype selon la revendication 4, caractérisé en ce que le support (1) est constitué comme un pupitre présentant au moins une surface ayant une inclinaison convenant à la vision de près.

**Fig.1**

Jede größere Stadt hat mindestens

ein größeres Erholungsgebiet

**Fig.2**

ein größeres Erholungsgebiet

in der näheren Umgebung

**Fig.3**

Jede größere Stadt hat mindestens

ein größeres Erholungsgebiet

in der näheren Umgebung

**Fig.4**